# EUROPEAN PATENT SPECIFICATION

(11) **EP 1 469 738 B1**
(45) Date of publication and mention of the grant of the patent: **30.01.2008**
(21) Application number: 02792116.2
(22) Date of filing: 02.12.2002
(51) Int. Cl.: A23C 20/00, A23L 1/23, A23L 1/30, C12N 1/20, A23J 3/34, C12R 1/40, C12R 1/44, C12R 1/46, A23C 19/06, A23C 19/032, C12P 21/06, C12P 1/04

(54) **CHEESE FLAVOUR INGREDIENT AND METHOD OF ITS PRODUCTION**
KÄSEGESCHMACKSZUTAT UND IHRE HERSTELLUNG
SUBSTANCE AROMATIQUE DE FROMAGE ET PROCEDE DE FABRICATION

(30) Priority: 03.12.2001 NZ 51588101
(43) Date of publication of application: 27.10.2004
(73) Proprietor: Fonterra Co-Operative Group Limited, Auckland (NZ)
(72) Inventor: CROW, Vaughan, Leslie, Palmerston North (NZ); HAYES, Michelle, Palmerston North (NZ); CURRY, Brian, James, Palmerston North (NZ); SAMAL, Prabandha, Kumar, Palmerston North (NZ); SCHOFIELD, Rachel, Helen, Palmerston North (NZ); HOLLAND, Ross, Palmerston North (NZ)
(74) Representative: Walker, Ross Thomson
(86) International application number: PCT/NZ2002/000266
(87) International publication number: WO 2003/047358

(56) References cited:
- EP-A1- 0 137 536
- EP-A2- 0 508 701
- WO-A-02/085131
- WO-A1-99/35240
- GB-A- 930 107
- GB-A- 1 449 279
- US-A- 4 172 900
- US-A- 4 675 193
- US-A- 4 708 876
- US-A- 6 054 151
- DATABASE CA XP002980784 Database accession no. 106:83290 & JP 61 274 663 A 1986
- DATABASE WPI Week 197749, Derwent Publications Ltd., London, GB; AN 1977-87396Y, XP002980768 & JP 52 128 286 A (TANABE SEIYAKU KK) 1977

## Description

### Technical Field

This invention relates to a method of manufacturing a cheese flavour ingredient and an ingredient so prepared. It is also relates to bacterial strains useful in the preparation of such an ingredient.

### Background to the Invention

The expression "cheese flavour ingredient" means an ingredient from a protein source that is a component of a mixture of ingredients that have a cheese flavour. The cheese flavour ingredient may or may not itself have a cheese flavour.

Cheese is a biologically dynamic food in which flavour develops as a result of the activity of the enzymes and cultures that occur in the cheese. Flavour develops in the cheese as the enzymes and cultures trapped in the curd act on the milk substrates - caseins, fats, and residual lactose (Crow *et al,* 1993). The initial ripening reactions are hydrolytic, generating peptides and amino acids from milk caseins by the action of proteases and free fatty acids from milk fat by the action of esterases and lipases. The later reactions during cheese ripening are more complex. Amino acids, free fatty acids and lactate are the primary substrates and the flavour reactions involve, amongst other transformations, the dynamic interaction of deamination reactions, transferase reactions, and synthetic reactions (McSweeny *et al,* 2000).

Often cheese flavours take months and years to develop. Flavour development can be greatly accelerated using an enzyme modified cheese (EMC) process. In an EMC process, selected enzymes (proteases and lipases) are added to a cheese curd that is typically slurried with water. Intense flavours develop over a period of hours to days (Kilkawley *et al,* 1998). An EMC process is described in US patent 3,765,905. EMC processes often involve both proteolysis of dairy proteins and lipolysis of dairy fats. Examples of such EMC processes are disclosed in international patent application WO 99/63834, US patent 5,455, 051 and European Patent application EP 1 053 689.

WO 02/085131 reports a process for preparing a cheese-flavoured ingredient having a weak savoury flavour by fermentation of pasteurised reconstituted whole milk powder using Lactobacillus Bulgaricus, Enterococcus faecallis and Macrococcus caseolyticus

However, often these flavour enhancing processes amplify particular groups of flavourful compounds, such as short chain fatty acids, and peptides of a particular molecular weight and as a consequence, the products of these EMC processes lack the balanced flavour profile of a cheese that has been ripened naturally.

### Object of the Invention

It is an object of this invention to go some way towards overcoming this disadvantage or at least to offer the public a useful choice.

### Summary of the Invention

Accordingly, the invention may be said broadly to consist in a process for the manufacture of a cheese flavour ingredient which comprises the steps of:
(a) forming a fat protein in water mixture,
(b) adding a protease enzyme to said mixture to establish a proteolysis reaction,
(c) adding a bacterial culture to said mixture to establish a fermentation reaction, said bacterial culture comprising a physiologically acceptable bacterium capable of producing a cheese flavour ingredient selected from the group consisting of *Enterococcus, faecium* B9642, AGAL accession number NM 01/24754, *Enterococcus. faecium* B9645, AGAL accession number NM 01/24755, Enterococcus faecalis B9509, AGAL accession number NM 01/24757, Enterococcus casseliflavus B9518, AGAL. accession number NM 01/24753, *Staphylococcus simulans* B9646, AGAL accession -number NM 01/24756 and *Pseudomonas putida* 89647, AGAL accession number NM 01/2.4752, bacteria,
(d) maintaining said mixture under microaerophilic or anaerobic conditions at a temperature within the range of about 20-50°C at a pH within the range of about 5.0 to 8.0 for a time period of from about 20 to 100 hours, and
(e) terminating said reactions and recovering the cheese flavour ingredient produced.

Preferably, said protein comprises a dairy protein. More preferably, said dairy protein comprises casein. Preferably said mixture comprises a fat, protein and water emulsion. In a preferred embodiment said mixture comprises cheese and water.

Alternatively, said mixture comprises one or more cheese curd, ripened cheese, mature cheese, milk solids, reconstituted whole milk powder, milk protein concentrate, a casein, a milk protein or a non-dairy protein, milk fat or a non-dairy oil or fat.

Preferably said mixture comprises total solids in the range of 5% (w/w) to 50% (w/w) of the emulsion.

Preferably, said protease enzyme is a peptidase or a proteinase or a combination of a peptidase and a proteinase.

In one embodiment, said proteinase is protease A "Amano" (Amano Enzymes) and said peptidase is Neutrase (Novo Nordisk).

In another embodiment said proteinase is Flavorpro 192P (Biocatalysts) and said peptidase is Promod 215P (Biocatalysts).

In one embodiment the method comprises maintaining the emulsion at a temperature within the range of 20-60°C to continue said proteolysis reaction before beginning step (c). Preferably, said proteolysis reaction is conducted at a temperature of from 40-50°C. Most preferably, said proteolysis reaction is conducted at a temperature of 43 °C.

In one embodiment said proteolysis reaction is conducted for some 2 to 24 hours before beginning step (c). Most preferably, said proteolysis reaction is conducted for about 4 hours.

In one embodiment said proteolysis reaction is terminated by heating the reaction mixture to 80-100°C for from 3 to 30 minutes before beginning step (c). Most preferably, said proteolysis reaction is terminated by raising the temperature of the reaction mixture to about 93°C for about 15 minutes.

In one embodiment, said mixture is cooled after being heated to terminate said proteolysis reaction.

Preferably, further protease is added after said cooling step.

In one embodiment steps (b) and (c) are allowed to proceed at the same time.

Preferably, said fermentation reaction is conducted at a temperature of from 30 to 45°C.

Most preferably, said fermentation reaction is conducted at a temperature of 40°C.

Preferably, said fermentation reaction is conducted at a pH of from 6.3 to 6.5.

Preferably, said fermentation reaction is conducted for from 30 to 72 hours.

More preferably, said fermentation reaction is conducted for between about 50 and about 65 hours. Most preferably, said fermentation reaction is conducted for about 53 hours or about 64 hours.

In one embodiment cheese and/or salt may be added during formation of the mixture, where required, to provide a desired solid contents and salt level in any final product In another embodiment additional cheese and salt may be added immediately before said fermentation reaction is terminated. In another embodiment, cheese and/or salt may be added part way through said fermentation reaction.

Preferably, said fermentation reaction is terminated by heating to a range of 80°C to 100°C and holding for a time of 3 to 30 minutes.

Most preferably, said fermentation reaction is terminated by heating to a temperature of about 93°C and holding for a time of about 15 minutes.

In one embodiment salt is added to said reaction mixture after said fermentation reaction has been terminated.

In another embodiment, cheese is added to said reaction mixture after said fermentation reaction has been terminated.

In one embodiment, the cheese flavour ingredient produced by said fermentation reaction is dried.

Preferably, said ingredient is dried by spray drying.

One embodiment of the invention comprises a cheese flavour ingredient produced by the method of the invention. Another embodiment of the invention comprises a food product comprising a cheese flavour ingredient of the invention. Preferably said food product comprises a product selected from the group comprising natural cheese, processed cheese, analogue cheese, foods comprising natural cheese, processed cheese or analogue cheese, sauces, dips, snacks, biscuits, soups, pizza, and cheese and savoury flavoured foods.

The invention may also be said broadly to consist in the bacterial strain *Euterococcus faeciu*m B9642, AGAL accession number NMO1/24754.

Alternatively, the invention consists in the bacterial strain *Enterococcus faecium* B9645, AGAL accession number NMO1/24755.

In another embodiment said bacterium is *Enterococcus faecaelis* B9509, AGAL accession number NM01/24757.

In another embodiment said bacterium is *Enterococcus casseliflavus* B9518, AGAL accession number NMO1/24753.

In another embodiment said bacterium is *Staphylococcus simulans* B9646, AGAL accession number NM01/24756.

In another embodiment said bacterium is *Pseudomonas putida* B9647, AGAL accession number NM01/24752.

Another embodiment of the invention comprises a food product comprising a biologically pure culture of the invention.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

### Brief Description of the Figure

The invention may also be more fully understood by having reference to the accompanying drawing wherein:

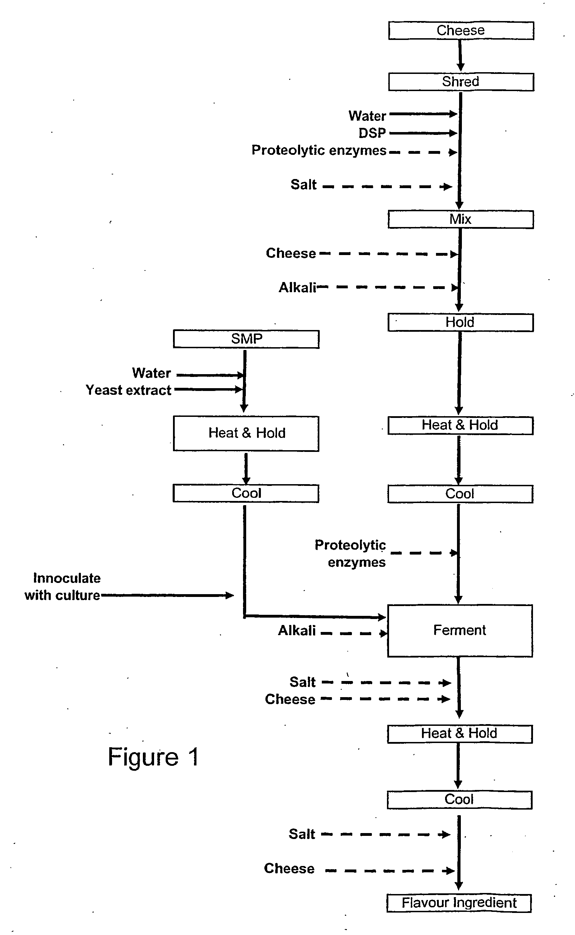

Figure 1 is a flow diagram of a preferred embodiment of the invention.

### Detailed Description of Preferred Embodiments

In the preferred embodiment a protein in water mixture is prepared. Preferably, the protein is a dairy protein source such as cheese. Preferably a cheese, such as a Cheddar or Gouda, is shredded and water is added together with an emulsifying agent such as disodium phosphate (DSP) to form an emulsion. Preferably, the total solids in the mixture range between 5% (w/w) to 50% (w/w).

The preferred emulsifying salt is disodium phosphate (DSP). It is added until its concentration is up to 5% w/w. A preferred concentration is around 1.2% w/w. Other emulsifying food grade salts such as other phosphates and citrate salts may be used.

In addition, food grade emulsifying gums or other food grade emulsifying compounds may be used to assist in the formation of the emulsion.

If necessary, the pH of the mixture may be adjusted to within a range of pH 6.3 to 6.5 by adding a food grade base (alkali).

The reaction mixture is preferably heated to inactivate microbes in the mixture. A preferred temperature is about 93°C for approximately 15 minutes. The total time elapsed from initiating the process to this first heating step is preferably about 60 minutes to ensure adequate mixing and pH stabilisation.

The mixture is preferably then cooled to about 40°C.

The protease enzymes may be added before or after the heating step. In a preferred embodiment, about 0.1% (w/w) of the protease enzymes are added to the mixture. If the protease enzymes are added before the heating step then preferably the heating step also serves to terminate proteolysis.

The preferred protease enzymes which are added to the mixture are a blend of proteinase and peptidase enzymes. Protease A "Amano" (Amano Enzymes) and Neutrase (Novo Nordisk) are exemplary of a blend which can be employed. Another blend is Flavorpro 192P (Biocatalysts) protease and Promod 215P (Biocatalysts) peptidase.

In one embodiment, once the proteolytic enzymes are incorporated, the mixture is preferably held at a reaction temperature of about 20 to 60°C, preferably about 40°C, for about at least 10 minutes until the mixture is readily stirrable.

In another embodiment the proteolysis reaction and the fermentation reaction are allowed to proceed at the same time.

A bacterial culture of *Enterococcus faecium* B9642 (as defined below) is added to the mixture and cultured with minimal agitation, to minimise air entrapment. Strict anaerobic conditions are not required as the metabolic activity of the added enterococcal culture will ensure that dissolved oxygen levels are minimal during the course of the fermentation. However, the reaction is carried out under microaerophilic or anaerobic conditions.

A fermentation reaction is thereby established. The temperature is maintained at approximately 20 to 50°C, preferably about 40°C. If necessary the pH is maintained within a range of 6.3 to 6.5 by adding a food grade base. This may be done continuously or at intervals up to about 12 hours.

The fermentation reaction is continued for 20 to 100 hours until the desired ingredient has been produced. A preferred fermentation time is about 50 to 65 hours, about 50 to 54 hours or about 60 to 64 hours.

In some cases it is desirable to adjust the solids content of the fermentation mixture by adding additional starting material, such as grated cheese prior to terminating the fermentation. Salt may be added at the same time or at any other time during the fermentation.

The fermentation step is terminated by heating the mixture. In one embodiment the proteolysis reaction is also terminated at this point. A preferred temperature is 93°C for approximately 15 minutes to inactivate the culture and its enzymes. Salt content is adjusted to give a final concentration of 5% in the aqueous phase. The solids content may be adjusted when desired by the addition of grated cheese.

The flavour ingredient is in the deactivated reaction mixture. This may be used directly or it may be dried by spray drying, for example, for further use.

The culture which is added to the ferment in the flow diagram of Figure 1 is preferably made up as follows. Skim milk powder (SMP) is reconstituted as a 10% aqueous solution to which 0.1% (w/w) yeast extract is added. The medium is then sterilised by heating to preferably 120°C for typically 15 minutes.

The medium is then cooled to 30 to 45°C prior to inoculation. A culture of the bacterium *Enterococcus faecium* B9642 is added under sterile inoculation conditions. The inoculated medium is incubated for 20 to 24 hours to allow for culture growth. During growth of the culture the pH is adjusted to maximise cell densities above 10⁹/ml. The prepared culture may either be stored frozen at -20°C or stored chilled at 5°C until required, or added immediately to the fermentation vessel at typically 4% (w/w) inoculation level to initiate the fermentation. Other means known in the art of producing a culture are also useful herein.

The flavour ingredient recovered after completion of the fermentation has a concentrated mixture of flavours (derived chiefly from the products of amino acid fermentations). A panel comprising practitioners experienced in assessing the taste attributes of cheeses and cheese flavour concentrates was used to assess the product diluted in a bland white sauce. The white sauce was made from mixing and heating 125g of sauce powder (5g salt, 120g standard white flour and 500g cream powder) blended with 450 g of water. The ingredient was usually diluted in the range of 1 to 20%, typically at 10%, to produce strong flavours that enable batch-to-batch comparison. The flavour was described as having a strong smear flavour, often described as a dirty socks flavour. The other main flavours described were cooked, salty, savoury flavours with sulphury notes. The flavour of the ingredient was compared with two other commercial ingredient flavour concentrates in different dilutions in the white sauce. The ingredient was found to be comparable to, but to have a higher potency than the two commercial concentrates derived by the forced maturation of smear ripened cheese curd.

The flavour ingredient can be used in a range of food applications either on its own or in combination with other flavouring agents. In general terms the ingredient is diluted sufficiently so that the smear flavour note is not detected but rather the ingredient provides flavour balance and enhances the impact of other flavour components. In most applications the flavour ingredient is used in the range of 0.1% to 2%. It may be used to enhance the flavour of natural cheese, processed cheese and analogue cheese foods. It can be used in sauces and dips, for example in a fondue recipe (14% Cheddar cheese, 23% cream, 47% milk, 9% butter, 4% flour, 2% other flavours) the 1% addition of the flavour ingredient improves the savoury and cheesy taste. It can be used in other foods requiring a cheese like flavour component such as snacks, biscuits, soups, pizza or other cheese or savoury flavoured food.

The bacterium used in the fermentation step according to the invention is of one of the genera *Enterococcus, Staphylococcus* and *Pseudomonas.*

*Enterococcus* is the preferred genus for this fermentation. The genus *Enterococcus* was proposed originally for gram positive diplococci of intestinal origin (Schleifer and Kilpper-Balz, 1984). The genus can be distinguished from other gram-positive, catalase-negative cocci by their ability to grow between 10°C and 45°C, in 6.5% NaCl, at high pH (pH 9.6) (Hardie *et al,* 1997). While some strains of *Enterococcus* have been associated with opportunistic secondary infections in humans (Franz *et. al.,* 1999), these are of a type not found in foods. In fact, several strains are used as probiotics to confer health benefits when they are consumed and many strains are used for the positive contribution they make to food fermentations that include cheeses and other fermented milk products (Franz *et al,* 1999).

Enterococci occur in a number of cheese varieties but are particularly associated with the cheese produced in southern Europe. They may occur in numbers ranging from 10⁴ - 10⁶ CFU/g in some ripened cheeses, including Emmental. The predominant enterococcal isolates from cheese are *E. faecalis* and *E. faecium.* The beneficial contribution of *Enterococci* to flavour in cheese has been attributed to the metabolic activity of the cultures, particularly the spectrum of proteolytic enzymes (Centeno *et al,* 1996) and esterase enzymes (Tsakalidou *et al,* 1993) and the wide ranging aromatic flavour compounds produced by the cultures.

*Enterococcus faecium* B9642 is a preferred bacterial strain. It is stored in the culture collection of the Dairy Research Institute, Palmerston North, New Zealand as culture number B9642. It is also deposited in the culture collection of the Australian Government Analytical Laboratory (AGAL), 1 Suakin Street, Pymbal, NSW 2073, Australia as accession number NM01/24754.

Enterococci are Gram-positive, catalase negative cocci mostly in pairs or chains. They produce lactic acid as the major end product of fermentation and do not form gas. Enterococci can be distinguished from other streptococci by their ability to grow at 10 and 45°C, grow in 6.5% sodium chloride, grow at pH 9.6 and to survive heating at 60°C for 30 minutes. They posses group D antigen (Lancefield serological typing scheme).

Further characteristics of the *Enterococcus* strains used in this invention are set out below in Table 1.

**Table 1. Preferred Enterococcus strains have the following characteristics.**

| **Test** | | ***E. faecium* B9642** | ***E. faecium* B9645** | ***E. faecalis* B9509** | ***E. casseliflavus* B9518** |
|---|---|---|---|---|---|
| Microscopic appearance | | oval cocci | oval cocci | oval cocci | oval cocci |
| Gram stain | | + | + | + | + |
| Catalase | | - | - | - | - |
| Growth at: | 10°C | + | + | + | + |
| | 45°C | + | + | + | + |
| | 55°C | - | - | - | - |
| NH₃ from arginine | | + | + | + | nd* |
| Acid from: | L-Arabinose | ± | ± | - | + |
| | D-Arabinose | - | - | - | - |
| | Glucose | + | + | + | + |
| | Lactose | + | + | + | + |
| | Maltose | + | + | + | + |
| | Mannose | + | + | + | + |
| | Melezitose | + | + | + | ± |
| | Melibiose | - | - | + | + |
| | Raffinose | - | - | + | + |
| | Ribose | + | + | + | + |
| | Sorbitol | - | - | + | + |
| | Sucrose | + | + | + | - |
| | Trehalose | + | + | + | + |

| | | | | | |
|---|---|---|---|---|---|
| * nd = not determined | | | | | |

A culture of *Staphylococcus* has also been used in this invention. Staphylococci are Gram-positive, catalase positive, facultative anaerobic, non-motile cocci, 0.5 - 1.0 µm in diameter, dividing in more than one plane to form pairs and clusters. Staphylococci can be distinguished from other micrococcaceae by their ability to grow anaerobically and being oxidase negative. The major end product of fermentation anaerobically is lactate. Characteristics of the S. *simulans* B9646 strain are set out in Table 2 below.

**Table 2. Characteristics of S. simulans B9646.**

| **Test** | | ***S. simulans* B9646** |
|---|---|---|
| Microscopic appearance | | Cocci |
| Gram stain | | + |
| Catalase | | + |
| Growth at: | 10°C | - |
| | 15°C | + |
| | 45°C | + |
| | 55°C | - |
| NH₃ from arginine | | + |
| Acid from: | Fructose | + |
| | Glucose | + |
| | Lactose | + |
| | Maltose | - |
| | Mannitol | + |
| | Mannose | + |
| | Raffinose | - |
| | Ribose | - |
| | Sucrose | - |
| | Trehalose | + |
| | Turanose | - |

A culture of *Pseudomonas* has also been used in this invention. Pseudomonads are Gram-negative, catalase positive, usually oxidase positive, straight or curved rods motile by polar flagella. Their metabolism is respiratory, never fermentative, using oxygen as the terminal electron acceptor. Species in the genus *Pseudomonas* can be distinguished from other members of the pseudomonadaceae family because they do not require growth factors, do not grow at pH 3.6, do not form flocks with dendritic outgrowths and do not produce xanthomonadins. The characteristics of *P. putida* 9647 are set out in Table 3 below.

**Table 3. Characteristics of P. putida 9647.**

| **Test** | | ***P. putida* B9647** |
|---|---|---|
| Microscopic appearance | | rod |
| Gram stain | | - |
| Oxidase | | + |
| Growth at: | 4°C | + |
| | 37°C | + |
| | 45°C | - |
| NH₃ from arginine | | + |
| Acid from: | D-Arabinose | - |
| | Citrate | + |
| | Fructose | + |
| | Gluconate | + |
| | Glucose | + |
| | Maltose | + |
| | Mannitol | + |
| | Mannose | + |
| | Rhamnose | - |
| | Sucrose | - |
| | Trehalose | - |

The deposit details for the strains in each of Tables 1 to 3 are set out in Table 4.

**Table 4. Bacterial Strains and Deposit Details.**

| **Strain** | **AGAL Accession No.** | **Date** |
|---|---|---|
| *Enterococcus faecium* B9642 | NM01/24754 | 23/11/2001 |
| *Enterococcus faecium* B9645 | NM01/24755 | 23/11/2001 |
| *Enterococcus faecalis* B9509 | NM01/24757 | 23/11/2001 |
| *Enterococcus casseliflavus* B9518 | NM01/24753 | 23/11/2001 |
| *Staphylococcus simulans* B9646 | NM01/24756 | 23/11/2001 |
| *Pseudomonas putida* B9647 | NM01/24752 | 23/11/2001 |

The invention consists in the foregoing and also envisages constructions of which the following gives examples.

### Example 1

A protein in water mixture comprising a cheddar cheese was formed and then incubated with protease enzymes in a first incubation and then incubated with protease enzymes and a culture of *Enterococcus faecium* B9642 in a second incubation.

Ingredients for the first incubation: 500 kg of grated commercial New Zealand Cheddar cheese; 12 kg of food grade disodium phosphate dissolved in 50 L hot water; 600 g proteolytic enzymes (protease A "Amano" and Neutrase in equal amounts) dissolved in 10 L water at 43°C; 250L water at 43 to 45°C; 50% (w/w) solution of food grade NaOH.

Ingredients for the second incubation: 400 kg grated Cheddar cheese; 600 g proteolytic enzymes (protease A "Amano" and Neutrase in equal amounts) dissolved in 5 L sterile water; 26kg of *Enterococcus faecium* B9642 culture; 50% (w/w) solution of food grade NaOH; 19.5 kg cheese salt.

### First incubation:

1. The water was placed in a tank at a temperature of about 53°C so addition of the cold cheese would bring it down to about 43°C. The dissolved disodium phosphate was added and mixed into the water. Approximately half the grated cheese was added while stirring over about 15 to 20 minutes.
2. Freshly made protease solution was added, the mixture was stirred for about 5 minutes and then the remaining grated cheese added over about 10 minutes. The temperature was set to 43°C and the mixture incubated for about 4 hours. After about 3.5 hours NaOH (2.4 L) was added to take the pH to 6.5.
3. After 4 hours incubation at 43°C, the solution was heated to about 93°C and held for 15 minutes before cooling to about 40°C.

### Second incubation:

4. A further 600 mL NaOH solution was added to the mixture to take the pH to about 6.4-6.6. The proteolytic enzyme solution and 26 kg of the B9642 culture was added. The reaction mixture was held at 40°C with gentle stirring for about 53 hours. The pH was checked and adjusted to 6.4 to 6.6 if necessary.
5. At the end of the 53 hour incubation the grated cheese and cheese salt was added with mixing and the mixture heated to 93°C and held for 15 minutes.
6. The ingredient was packed at 85°C in 20 L pails with plastic liners.

### Example 2

A protein in water mixture comprising a Gouda cheese source was formed in a first incubation then incubated with protease enzymes and a culture of *Enterococcus faecium* B9642 in a second incubation.

Ingredients for the first incubation: 440 kg of grated brine salted commercial New Zealand Gouda cheese; 10.5 kg disodium phosphate dissolved in 75 L hot water; 3 kg of cheese salt; 325 L water at 43 to 45°C; 50% (w/w) solution of NaOH.

Ingredients for the second incubation; 760 kg grated Cheddar cheese; 1320 g proteolytic enzymes (protease A "Amano" and Neutrase in equal amounts) dissolved in 5 L sterile water; 26 kg of B9642 culture; 50% (w/w) solution of NaOH; 37 kg cheese salt.

### First incubation

1. The water was placed in a tank at about 53°C so the cold cheese would bring it down to about 43°C. The dissolved disodium phosphate was added with stirring following by approximately half the grated cheese over about 15 to 20 minutes. The mixture was stirred throughout the process.
2. The remaining grated cheese was added over 10 minutes. The temperature was set to about 43°C and the mixture incubated for 30 minutes. During this incubation, the cheese salt and 3.5 L NaOH (pH stable at 6.4 - 6.6) were added.
3. The solution was heated up to about 93°C and held for about 15 mins before cooling to about 37°C.

### Second incubation

4. The pH was adjusted to pH 6.4-6.6 if necessary and the temperature set to about 40°C. The proteolytic enzyme solution was added and the mixture stirred for about 10 minutes. Then 26 kg of B9642 was added and the mixture held at about 40°C with intermittent stirring for 64 hours. At end of the 64 hour incubation the grated cheese and cheese salt was added with mixing. Then the reaction mixture was heated to 93°C and held for 15 minutes.
5. The ingredient was packed in 20 L pails with plastic liners at 85°C.

The final flavour ingredient from each example had the same sensory profile described above (a strong smear flavour, often described as a dirty socks flavour; other main flavours described were cooked, salty, savoury flavours with sulphury notes) when evaluated in a white sauce application as described above.

### References

Centeno J A, Menendez S, Rodriguez-Otero J L (1996). Main microbial flora present as natural starters in Cebreiro raw cow's-milk cheese (Northwest Spain). International Journal of Food Microbiology, 33, 307-313.
Crow V L, Coolbear T, Holland R, Pritchard G G, Martley F G (1993). Starters as finishers: starter properties relevant to cheese ripening. International Dairy Journal, 3, 423-460.
Franz CMAP, Holzapfel WH and Stiles ME (1999). Enterococci at the crossroads of food safety? International Journal of Food Microbiology, 47, 1-24.
Hardie J M and Whiley R A (1997). Classification and overview of the genera Streptococcus and Enterococcus. Journal of Applied Microbiology Symposium supplement, 83, 1S-11S.
Kilkawley K N, Wilkinson M G and Fox P F (1998). Enzyme modified cheese review. International Dairy Journal, 8, 1-10.
McSweeny P L H and Sousa M J (2000). Biochemical pathways for the production of flavour compounds in cheeses during ripening: A review. Lait, 80, 293-324.
Schleifer K H and Kilpper-Balz R (1984). Transfer of Streptococcus faecalis and Streptococcus faecium to the genus Enterococcus nom. rev. as Enterococcus faecalis comb. nov. and Enterococcus faecium comb. Nov. International Journal of Systematic Bacteriology, 34,31-34.
Tsakalidou E, Manolopoulou E, Tsilibari V, Georgalaki M, and Kalanzopolous G (1993). Esterolytic activities of Enterococcus durans and Enterococcus faecium strains isolated from Greek cheese. Netherlands Milk and Dairy Journal, 47, 145-150.

## Claims

1. A method for the manufacture of a cheese flavour ingredient comprising the steps of:
(a) forming a protein in water mixture,
(b) adding a protease enzyme to said mixture to establish a proteolysis reaction,
(c) adding a bacterial culture to said mixture to establish a fermentation reaction, said bacterial culture comprising a physiologically acceptable bacterium capable of producing a cheese flavour ingredient selected from the group consisting of *Enterococcus, faecium* B9642, AGAL accession number NM 01/24754, *Enterococcus, faecium* B9645, AGAL accession number NM 01/24755, Enterococcus faecalis B9509, AGAL accession number NM 01/24757, Enterococcus casseliflavus B9518, AGAL accession number NM 01/24753, *Staphylococcus simulans* B9646, AGAL accession number NM 01/24756 and *Pseudomonas putida* B9647, AGAL accession number NM 01/24752, bacteria,
(d) maintaining said mixture under microaerophilic or anaerobic conditions at a temperature within the range of 20-50°C at a pH within the range of 5.0 to 8.0 for a time period from 20 to 100 hours, and
(e) terminating said reactions and recovering the cheese flavour ingredient produced.

2. A method as claimed in Claim 1 wherein said mixture is selected from mixtures comprising:
(a) a fat, protein and water emulsion;
(b) cheese and water; and
(c) one or more dairy protein, casein, cheese curd, ripened cheese, mature cheese, milk solids, reconstituted whole milk powder, milk protein concentrate, casein, milk protein, non-dairy protein milk fat and a non-dairy oil or fat.

3. A method as claimed in Claim 1 wherein said mixture comprises total solids in the range of 5% (w/w) to 50% (w/w) of the mixture.

4. A method as claimed in Claim 1 wherein said protease enzyme comprises a peptidase or a proteinase or a combination of a peptidase and a proteinase.

5. A method as claimed in Claim 1 wherein comprising maintaining said emulsion at a temperature within the range of 20-60°C to continue said proteolysis reaction before beginning step (c).

6. A method as claimed in Claim 5 wherein said proteolysis reaction is conducted for 2 to 24 hours.

7. A method as claimed in Claim 1 wherein said proteolysis reaction is terminated before beginning step (c).

8. A method as claimed in Claim 1 wherein said mixture is cooled after heated to terminate said proteolysis reaction and wherein further protease is optionally added after cooling step.

9. A method as claimed in Claimed 1 wherein said steps (b) and (c) are allowed to proceed at the same time.

10. A method as claimed in Claim 1 wherein said fermentation reaction is conducted at a temperature of from 30 to 45°C

11. A method as claimed in Claim 1 wherein fermentation reaction is conducted at a pH of from 6.3 to 6.5.

12. A method as claimed in Claim 1 wherein fermentation reaction is conducted for from 30 to 72 hours.

13. A method as claimed in Claim 1 wherein cheese, salt or both cheese and salt are added before, during or after step (d).

14. A method as claimed in Claim 1 comprising a further step wherein the cheese flavour ingredient is dried.

15. A cheese flavour ingredient produced by the method claimed in Claim 1.

16. A food product comprising cheese flavour ingredient as claimed in Claim 15.

17. A biologically pure culture of *Enterococcus faecium* B9642, AGAL accession number NM01/24754.

18. A biologically pure culture of *Enterococcus faecium* B9645, AGAL accession number NM01/24755; *Enterococcus faecaelis* B9509, AGAL accession number NM01/24757; *Enterococcus casseliflavus* B9518, AGAL accession number NM01/24753; *Staphylococcus simulans* B9646, AGAL accession number NM01/24756; or *Pseudomonas putida* B9647, AGAL accession number NM01/24752.

19. A food product comprising a biologically pure culture as claimed in Claim 17 or 18.

## Patentansprüche

1. Verfahren zur Herstellung einer Käsegeschmackszutat mit folgenden Schritten:
(a) ein Protein wird in einer Wassermischung gebildet,
(b) ein Proteaseenzym wird der Mischung zugefügt, um eine Proteolysereaktion hervorzurufen,
(c) eine Bakterienkultur wird der Mischung zugefügt, um eine Fermentationsreaktion herbeizuführen, wobei diese Bakterienkultur ein physiologisch annehmbares Bakterium aufweist, das eine Käsegeschmackszutat erzeugen kann und das aus der Gruppe ausgewählt ist, die aus den Bakterien *Enterococcus faecium* B9642, AGAL-Zugriffsnummer NM 01/24754, *Enterococcus faecium* B9645, AGAL-Zugriffsnummer NM 01/24756, *Enterococcus faecalis* B9509, AGAL-Zugriffsnummer NM 01/24757, *Enterococcus cassellflavus* B9518, AGAL-Zugriffsnummer NM 01/24753, *Staphyllococcus simulans* B9646, AGAL-Zugriffsnummer NM 01/24756 und *Pseudomonas putida* B9647, AGAL-Zugriffsnummer NM 01/24752, besteht,
(d) diese Mischung wird unter mikroaerophilen oder anaeroben Bedingungen bei einer Temperatur im Bereich von 20-50°C und einem pH-Wert im Bereich von 5,0-8,0 für eine Zeitdauer von 20-100 Stunden aufrecht erhalten und
(e) diese Reaktionen werden beendet, und die erzeugte Käsegeschmackszutat wird ausgeleitet.

2. Verfahren nach Anspruch 1,
**dadurch gekennzeichnet,**
**dass** diese Mischung aus Mischungen ausgewählt ist, die Folgendes umfassen:
(a) eine Fett-, Protein- und Wasseremulsion,
(b) Käse und Wasser und
(c) ein oder mehrere milchwirtschaftliche Proteine, Kasein, Käsequark, abgelagerter Käse, reifer Käse, Milchfeststoffe, wiederhergestelltes, ganzes Milchpulver, Milchproteinkonzentrat, Kasein, Milchprotein, nicht milchwirtschaftliches Proteinmilchfett und ein nicht milchwirtschaftliches Öl oder Fett.

3. Verfahren nach Anspruch 1,
**dadurch gekennzeichnet,**
**dass** diese Mischung Gesamtfeststoffe im Bereich von 5-50 Gew.-% der Mischung aufweist.

4. Verfahren nach Anspruch 1,
**dadurch gekennzeichnet,**
**dass** das Proteaseenzym eine Peptidase oder eine Proteinase oder ein Kombination aus einer Peptidase oder einer Proteinase aufweist.

5. Verfahren nach Anspruch 1,
**dadurch gekennzeichnet,**
**dass** diese Emulsion bei einer Temperatur im Bereich von 20-60°C aufrecht erhalten wird, um die genannte Proteolysereaktion vor dem Schritt (c) fortzusetzen.

6. Verfahren nach Anspruch 5,
**dadurch gekennzeichnet,**
**dass** diese Proteolysereaktion 2-24 Stunden lang durchgeführt wird.

7. Verfahren nach Anspruch 1,
**dadurch gekennzeichnet,**
**dass** diese Proteolysereaktion vor dem Beginn des Schritts (c) beendet wird.

8. Verfahren nach Anspruch 1,
**dadurch gekennzeichnet,**
**dass** diese Mischung nach ihrem Erhitzen gekühlt wird, um die genannte Proteolysereaktion zu beenden, und dass ferner Protease nach dem Kühlschritt wahlfrei zugefügt wird.

9. Verfahren nach Anspruch 1,
**dadurch gekennzeichnet,**
**dass** die Schritte (b) und (c) zur gleichen Zeit vorgenommen werden können.

10. Verfahren nach Anspruch 1,
**dadurch gekennzeichnet,**
**dass** diese Fermentationsreaktion in einem Temperaturbereich von 30-45°C durchgeführt wird.

11. Verfahren nach Anspruch 1,
**dadurch gekennzeichnet,**
**dass** diese Fermentationsreaktion in einem pH-Wertbereich von 6,3-6,5 durchgeführt wird.

12. Verfahren nach Anspruch 1,
**dadurch gekennzeichnet,**
**dass** diese Fermentationsreaktion in einem Zeitdauerbereich von 30-72 Stunden durchgeführt wird.

13. Verfahren nach Anspruch 1,
**dadurch gekennzeichnet,**
**dass** Käse, Salz oder sowohl Käse als auch Salz vor dem Schritt (d), während dieses Schrittes oder nach diesem Schritt zugefügt wird.

14. Verfahren nach Anspruch 1,
**dadurch gekennzeichnet,**
**dass** ein weiterer Schritt vorgesehen ist, in dem die Käsegeschmackszutat getrocknet wird.

15. Käsegeschmackszutat, die nach dem im Anspruch 1 beanspruchten Verfahren hergestellt ist.

16. Nahrungsmittelprodukt, das eine Käsegeschmackszutat nach Anspruch 15 aufweist.

17. Biologisch reine Kultur des Bakteriums *Enterococcus faecium* B9642, AGAL-Zugriffsnummer NM 01/24754.

18. Biologisch reine Kultur des Bakteriums *Enterococcus faecium* B9645, AGAL-Zugriffsnummer NM 01/24755, *Enterococcus faecalis* B9509, AGAL-Zugriffsnummer NM 01/24757, *Enterococcus cassellflavus* B9518, AGAL-Zugriffsnummer NM 01/24753, *Staphyllococcus simulans* B9646, AGAL-Zugriffsnummer NM 01/24756 oder *Pseudomonas putida* B9647, AGAL-Zugriffsnummer NM 01/24752.

19. Nahrungsmittelprodukt aus einer biologisch reinen Kultur nach Anspruch 17 oder 18.

## Revendications

1. Procédé pour la fabrication d'un ingrédient aromatique de fromage comprenant les étapes de :
(a) former une protéine dans un mélange d'eau,
(b) ajouter une enzyme protéase audit mélange pour établir une réaction protéolytique,
(c) ajouter une culture bactérienne audit mélange pour établir une réaction de fermentation, ladite culture bactérienne comprenant une bactérie physiologiquement acceptable pouvant produire un ingrédient aromatique de fromage choisi parmi le groupe constitué des bactéries *Enterococcus, faecium* B9642, AGAL numéro d'accès NM 01/24754, *Enterococcus, faecium* B9645, AGAL numéro d'accès NM 01/24755, Enterococcus faecalis B9509, AGAL numéro d'accès NM 01/24757, Enterococcus casseliflavus B9518, AGAL numéro d'accès NM 01/24753, *Staphylococcus simulans* B9646, AGAL numéro d'accès NM 01/24756 et *Pseudomonas putida* B9647, AGAL numéro d'accès NM 01/24752,
(d) maintenir ledit mélange sous des conditions microaérophiles ou anaérobies à une température dans la plage de 20-50°C à un pH dans la plage de 5,0 à 8,0 pendant une période de temps de 20 à 100 heures, et
(e) terminer lesdites réactions et récupérer l'ingrédient aromatique de fromage produit.

2. Procédé tel que revendiqué dans la revendication 1, dans lequel ledit mélange est choisi à partir de mélanges comprenant :
(a) une émulsion d'eau et protéine, grasse;
(b) du fromage et de l'eau ; et
(c) un ou plusieurs de protéine de produits laitiers, de caséine, de caillé de fromage, de fromage affiné, de fromage mûr, d'extrait du lait, de lait en poudre entier reconstitué, de concentré de protéines de lait, de caséine, de protéine de lait, de matière grasse de lait de protéine de produits non laitiers et d'huile ou de graisse de produits non laitiers.

3. Procédé tel que revendiqué dans la revendication 1, dans lequel ledit mélange comprend des matières solides totales dans la plage de 5% (p/p) à 50% (p/p) du mélange.

4. Procédé tel que revendiqué dans la revendication 1, dans lequel ladite enzyme protéase comprend une peptidase ou une protéinase ou une combinaison d'une peptidase et d'une protéinase.

5. Procédé tel que revendiqué dans la revendication 1, dans lequel il consiste à maintenir ladite émulsion à une température dans la plage de 20-60°C pour poursuivre ladite réaction de protéolyse avant de commencer l'étape (c).

6. Procédé tel que revendiqué dans la revendication 5, dans lequel ladite réaction de protéolyse est menée pendant 2 à 24 heures.

7. Procédé tel que revendiqué dans la revendication 1, dans lequel ladite réaction de protéolyse se termine avant le début de l'étape (c).

8. Procédé tel que revendiqué dans la revendication 1, dans lequel ledit mélange est refroidi après chauffage pour terminer ladite réaction de protéolyse et dans lequel une autre protéase est éventuellement ajoutée après l'étape de refroidissement.

9. Procédé tel que revendiqué dans la revendication 1, dans lequel on laisse se dérouler en même temps lesdites étapes (b) et (c).

10. Procédé tel que revendiqué dans la revendication 1, dans lequel ladite réaction de fermentation est menée à une température de 30 à 45°C.

11. Procédé tel que revendiqué dans la revendication 1, dans lequel la réaction de fermentation est menée à un pH de 6,3 à 6,5.

12. Procédé tel que revendiqué dans la revendication 1, dans lequel la réaction de fermentation est menée pendant 30 à 72 heures.

13. Procédé tel que revendiqué dans la revendication 1, dans lequel du fromage, du sel ou à la fois du fromage et du sel sont ajoutés avant, pendant ou après l'étape (d).

14. Procédé tel que revendiqué dans la revendication 1, comprenant une autre étape dans laquelle l'ingrédient aromatique de fromage est séché.

15. Ingrédient aromatique de fromage produit par le procédé revendiqué dans la revendication 1.

16. Produit alimentaire comprenant un ingrédient aromatique de fromage tel que revendiqué dans la revendication 15.

17. Culture biologiquement pure de *Enterococcus faecium* B9642, AGAL numéro d'accès NM01/24754.

18. Culture biologiquement pure de *Enterococcus faecium* B9645, AGAL numéro d'accès NM01/24755 ; *Enterococcus faecaelis* B9509, AGAL numéro d'accès NM01/24757 ; *Enterococcus casseliflavus* B9518, AGAL numéro d'accès NM01/24753 ; *Staphylococcus simulans* B9646, AGAL numéro d'accès NM01/24756 ; ou *Pseudomonas putida* B9647, AGAL numéro d'accès NM01/24752.

19. Produit alimentaire comprenant une culture biologiquement pure telle que revendiquée dans la revendication 17 ou 18.
